# EUROPEAN PATENT SPECIFICATION

(11) **EP 2 640 876 B1**
(45) Date of publication and mention of the grant of the patent: **01.03.2017**
(21) Application number: 11840749.3
(22) Date of filing: 17.11.2011
(51) Int. Cl.: B01L 3/00, G01N 33/543

(54) **METHOD FOR PRINTING IN-WELL CALIBRATION FEATURES**
VERFAHREN ZUM AUSDRUCKEN IN-WELL KALIBRIERFUNKTIONEN
PROCÉDÉ PERMETTANT D'IMPRIMER DES CARACTÉRISTIQUES DE CALIBRAGE DANS UN PUITS

(30) Priority: 17.11.2010 US 414663 P
(43) Date of publication of application: 25.09.2013
(73) Proprietor: Aushon Biosystems, Billerica, MA 01821 (US)
(72) Inventor: HONKANEN, Peter, Concord, MA 01742 (US); BURNS, Christine, A., Wellesley, MA 02481 (US)
(74) Representative: Stainthorpe, Vanessa Juliet
(86) International application number: PCT/US2011/061184
(87) International publication number: WO 2012/068372

(56) References cited:
- WO-A1-2004/104230
- US-A1- 2003 198 967
- US-A1- 2004 049 351
- US-A1- 2004 265 923
- US-A1- 2007 184 494
- US-A1- 2007 259 366
- US-A1- 2008 032 281
- US-A1- 2010 075 864
- US-A1- 2010 093 557
- LING MICHAEL M ET AL: "MULTIPLEXING MOLECULAR DIAGNOSTICS AND IMMUNOASSAYS USING EMERGING MICROARRAY TECHNOLOGIES", EXPERT REVIEW OF MOLECULAR DIAGNOSTICS, FUTURE DRUGS, LONDON, GB, vol. 7, no. 1, 1 January 2007 (2007-01-01) , pages 87-98, XP009080968, ISSN: 1473-7159, DOI: 10.1586/14737159.7.1.87

## Description

### CROSS-REFERENCE TO RELATED APPLICATIONS

This application claims the benefit under 35 U.S.C. § 119(e) of U.S. Provisional Patent Application Serial No. 61/414663, filed on November 17, 2010, entitled METHOD OF AND SYSTEM FOR PRINTING IN-WELL CALIBRATION FEATURES.

### BACKGROUND

### Field of Invention

The present invention relates to preparation of assay substrates, and, more specifically, to methods for printing in-well calibration features onto assay substrates.

### Description of Related Art

An assay substrate is a surface upon which various chemical and/or biological analyses can be performed. Examples of an assay substrate include microarray plates, glass slides, and microtiter plates. A microtiter plate is a flat plate that has multiple "wells" formed in its surface. Each well can be used as a small test tube into which various materials can be placed to perform biochemical analyses. One illustrative use of microtiter plates includes an enzyme-linked immunosorbent assay (ELISA), which is a modem medical diagnostic testing technique.

Generally, in an ELISA, a capture antibody is printed in the bottom of a well in a microtiter plate. The capture antibody has specificity for a particular antigen for which the assay is being performed. A sample to be analyzed is added to the well containing the capture antibody, and the capture antibody "captures" or immobilizes the antigen contained in the sample. A detect antibody is then added to the well, which also binds and/or forms a complex with the antigen. Further materials are then added to the well which cause a detectable signal to be produced by the detect antibody. For example, when light of a specific wavelength is shone upon the well, the antigen/antibody complexes will fluoresce. The amount of antigen in the sample can be inferred based on the magnitude of the fluorescence. In another example, a compound can be added to the well that causes the detect antibody to emit light within a predetermined wavelength (e.g., 400-500 nm). This light can be read by a charged-coupled device (CCD) camera to measure the optical brightness of the emitted light.

During an ELISA, the absorbency, fluorescence, or electrochemical signal of the well can be measured and compared with a standard to more accurately determine the presence and quantity of the sample antigen. For example, a calibration feature with a known concentration of antigen can be placed in wells separate from the wells that receive antigen-containing patient samples. However, signal variability, such as fluorescence variability, in the different wells can decrease the accuracy of comparing results from separate wells.

Thus, a need exists for methods and systems to provide and improve accuracy and reliability in medical diagnostic testing techniques and other biochemical analyses. US 2003/198967 (Matson et al.) discloses a detection device directed to a universal binding ligand that is attached to a substrate. Multiple reactive materials, such as analyte sensors, e.g., a capture agent or an antibody, are attached to the universal binding ligand to create a template or an array. The template or array can subsequently be further reacted with an analytical sample.

### BRIEF DESCRIPTION OF THE DRAWINGS

Figures 1A-B show a cross-sectional side view and a top view, respectively, of two wells in a microtiter plate.
Figures 2A-C show a series of cross-sectional side views of a well in a microtiter plate during a known method of conducting an ELISA.
Figure 3 shows a method of preparing in-well calibration features in accordance with some embodiments.
Figures 4A-C show a series of cross-sectional side views of a well in a microtiter plate during a method of conducting an ELISA in accordance with some embodiments.
Figure 5 shows a cross-sectional side view of a well in a microtiter plate with a number of printed features in accordance with some embodiments.

### DETAILED DESCRIPTION

The invention provides a method for detecting an antigen in a sample, the method comprising:
printing a plurality of capture antibody features in a well of a testing substrate,
printing a calibration compound of known concentration on one of the capture antibody features in the well of the testing substrate,
wherein the calibration compound of known concentration is capable of binding to the capture antibody feature,
wherein at least one capture antibody feature in the well of the testing substrate does not have a calibration compound of known concentration printed onto the at least one capture antibody feature and is capable of binding to the antigen in the sample, and
adding the sample to the well of the testing substrate,
wherein the calibration compound and the antigen are each capable of binding to a same enzyme linked detect antibody.

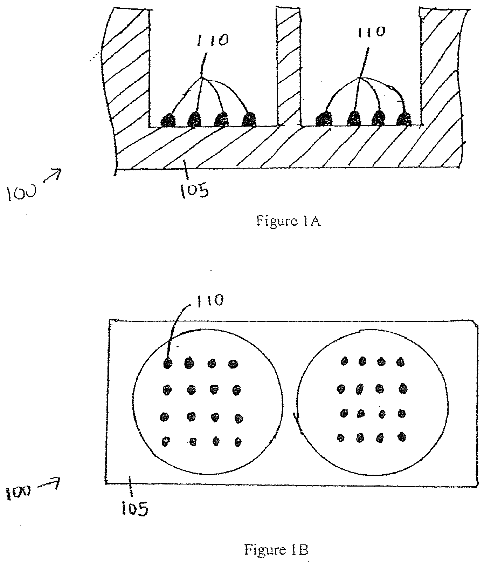

Figure 1A shows an illustration of a cross-sectional side view of two wells in a microtiter plate 100. In one illustrative implementation, the well substrate is formed of a polystyrene base 105. Other potential substrate materials include, but are not limited to, nitrocellulose, glass, and other plastic materials. Figure 1B shows an illustration of a top view of two wells in a microtiter plate 100. During the preparation of a microtiter plate for use in biochemical analysis, many different capture antibody "features" 110 are printed in the well and adhere to the polystyrene base 105. As used herein, "features" can have different shapes, such as, for example, a rounded shape. The assay substrate can be, for example, a 96-well microtiter plate. The features can be, for example, about 300 µm to about 500 µm in diameter.

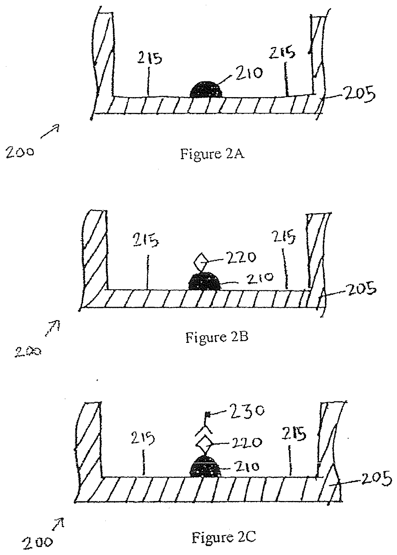

Figures 2A-C show a series of cross-sectional side views 200 of a well 205 during a known method of conducting an ELISA. After the capture antibody feature 210 has been printed onto the bottom of the well 205, a blocking material is added to the well to block plate binding sites 215 that remain on the plate 200. This prevents non-selective binding of sample antigens to the base of the well during the ELISA, which would give false readings. Second, an antigen-containing sample is added to the well. Figure 2B shows the antigen 220 binding to the capture antibody feature 210. Third, the well is washed so that unbound antigen is removed. Fourth, enzyme-linked detect antibodies are added. Figure 2C shows the enzyme-linked detect antibody 230 binding to the antigen 220. The well is then washed so that unbound antibody-enzyme conjugates are removed. Next, a substance is applied which converts the enzyme into a detectable signal, such as a color, fluorescent, or electrochemical signal. Finally, the absorbency, fluorescence, or electrochemical signal of the well is measured and compared with a standard to determine the presence and quantity of the sample antigen. A standard can be generated by printing calibration features with a known concentration of antigen in wells that are separate from the wells that receive patient samples.

Such an approach involves comparing standard results and sample results from different wells. Signal variability, such as fluorescence variability, and well-to-well variability in the separate wells can decrease the accuracy and reliability of test results.

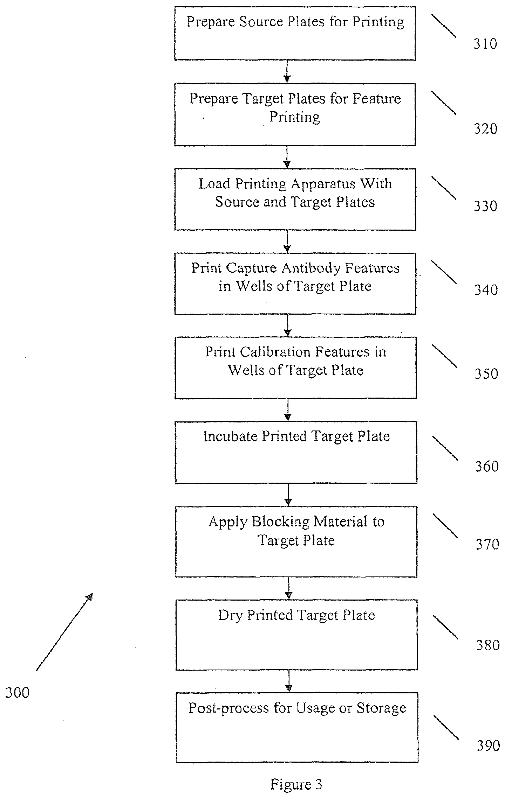

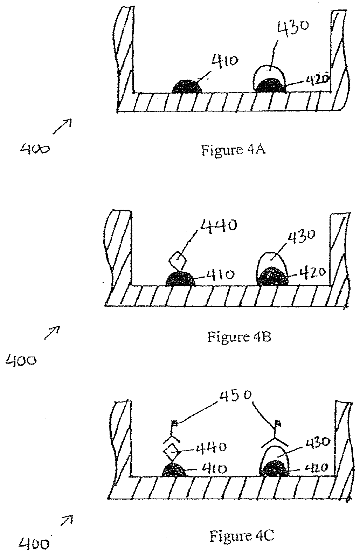

Figure 4A shows a cross-sectional side view of one plate well with two capture antibody features 410 and 420 and a calibration feature 430 printed on top of capture antibody feature 420. The calibration feature 430 is in the same well as the capture antibody feature 410 that will bind to the antigen-containing sample. Figure 3 shows a method 300 of printing in-well calibration features on a microtiter plate in accordance with some embodiments. Method 300 reduces or eliminates the inaccuracy that can result from signal variability and well-to-well variability by printing a calibration feature with a known amount of antigen in the same well as the capture antibody feature that binds to antigen-containing samples. Not only does method 300 reduce the variance of assay results, it also increases throughput, as all the wells of a plate can be used to analyze patient samples.

Suitable samples include proteomic samples such as, for example, from cell lysates, cell supernatants, plasma, serum, or other biological fluids. As used herein, a "target plate" is a plate that is to be prepared (e.g., printed, blocked, and processed for later usage) for a particular set of analyses. A "source plate" is a plate that has a supply of the material to be printed onto a target plate. For example, the wells of a source plate can be filled with various types of antibodies that are to be printed onto target plates. In accordance with method 300, the source plate is prepared for the printing process (step 310). This can include filling the wells of the source plate with the desired material to be printed onto the target plate. Next, the target plate is prepared for printing (step 320). This can include washing and/or performing other surface treatments to enable the material to be printed to properly adhere to the bottom surface of the plate well.

The source and target plates are then fit into a printing apparatus (e.g., a 2470 Arrayer available from Aushon Biosystems, Inc. of Billerica, MA) (step 330). Capture antibody features are printed in the wells of the target plate (step 340). Next, calibration features with a known concentration of an antigen are precisely printed onto the capture antibody features (step 350). The known concentration of an antigen ranges from the order of femtogram (10⁻¹⁵ g) per milliliter to milligram (10⁻³ g) per milliliter. Implementations of the invention using the 2470 Arrayer, independent of the arrayer's pin size, can achieve precise printing of the calibration features onto the capture antibody features, such that the positional misalignment between an outer edge of the calibration features and an outer edge of the capture antibody features is about 4 µm or less. Other implementations of the invention can tolerate positional misalignments between an outer edge of the calibration features and an outer edge of the capture antibody features of greater than about 4 µm, depending on the size of the features. For example, when printing features are in the range of about 120 µm to about 240 µm in diameter, positional misalignment between an outer edge of the calibration features and an outer edge of the capture antibody features of about 10 µm can be tolerated.

As described above, Figure 4A shows a cross-sectional side view of one plate well with two capture antibody features 410 and 420 and a calibration feature 430 printed on top of capture antibody feature 420. The calibration feature 430 is in the same well as the capture antibody feature 410 that will bind to the antigen-containing sample.

The printed target plate is incubated for a period of time (step 360), and a blocking material, which does not react to the capture antibody, is applied to the target plate using known methods (step 370). The blocking material adsorbs to the remaining binding surfaces of the plate and binds to antigens of non-specific interaction, thus reducing background signal. The printed target plate is then dried (step 380). In one illustrative implementation, a blocking material solution is applied to the surfaces of the bottoms of a plurality of wells in a microtiter plate via a spraying process, as described in U.S. Provisional Patent Application 61/372,552 entitled Method of and System for Applying Blocking Material to Assay Substrates, filed on August 11, 2010, the contents of which are incorporated by reference in its entirety.

During the spraying process, an airbrush (e.g., a Paasche Talon model TG0210) is used to apply the blocking material to the bottom surface of the well of the plate. During the spraying step, approximately 10 ml of a blocking material solution is sprayed over the entire surface of the plate. The blocking material is propelled by a compressed air source, *e.g.,* a standard air compressor that supplies clean and dry air, at a pressure of about 138 kPa (20 psig). The flow rate of the airbrush is set to about 10 ml/min. The application of the blocking material reduces or eliminates malformation and/or toppling of features during the addition of blocking material to the microtiter wells. The plates prepared according to the spraying process discussed herein have superior feature uniformity

The nozzle of the airbrush may be positioned about 15 cm (6 inches) from the surface of the plate, and the airbrush is swept across the entire surface while keeping the nozzle perpendicular to the surface of the plate. In other words, the center of the spray pattern is essentially normal to the surface of the plate. The spraying is continued at least until the level of blocking material in the well covers the printed features 530. After that level of blocking material is achieved, additional blocking material can be added by continuing the spraying process, or, optionally, additional blocking material can be added via micropipette, as described herein.

The application of the blocking material as described herein can be applied by-hand. In some implementations, the blocking can be applied by automated machinery. For example, after printing, incubating, and drying, the plate can be placed on a conveyor over which is mounted one or more spray nozzles. The rate of the conveyor is controlled to ensure adequate residence time of the plates within the spray pattern of the one or more nozzles. For example, if the total flow rate of all of the nozzles is about 10 ml/min, the conveyor speed can be controlled to provide that at least some portion of the surface of the plate is under the spray pattern for 1 minute. In another illustrative implementation, the plate can be held is a fixed position and an automated arm can direct one or more spray nozzles above the surface of the plate.

The specific operational parameters provided herein are merely illustrative, and other values are within the scope of the invention. For example, the blocking material flow rate can vary between 5-20 ml/min, the distance between the airbrush flow nozzle and the surface of the plate can vary between 2-41 cm (1-16 inches), and the air pressure can vary between 34-207 kPa (5-30 psig). It is understood that these ranges are merely illustrative and are not intended to be limiting.

The target plate is then processed for usage or storage using known methods (step 390). For example, the target plate can be incubated at about 4°C overnight. Alternatively, excess blocking material (e.g., the blocking material that has not bound to the bottom of the well) can be removed from the target plate, the plate can then be dried, and then the plate can be placed into a moisture-resistant package for storage. The disclosed method of printing in-well calibration features reduces or eliminates inaccuracy that can result from having the calibration feature printed in a separate well from the capture antibody feature that will bind to the antigen-containing sample. The disclosed method also increases throughput, as all the wells of a plate can be used to analyze patient samples.

The plates with in-well calibration features can then be used to conduct chemical and/or biological analyses, such as with an ELISA. Figure 4B shows a cross-sectional side view of the well after an antigen-containing sample has been added, and patient antigen 440 binds to the capture antibody 410. Next, enzyme-linked detect antibodies are added to the well. Figure 4C shows the enzyme-linked detect antibody 450 binding to the antigen 440 and calibration feature 430. A substance, such as a chemiluminescent substrate solution, is applied to convert the enzyme into a detectable signal. Finally, the signals are measured, and the presence and quantity of the sample antigen is determined using methods known in the art.

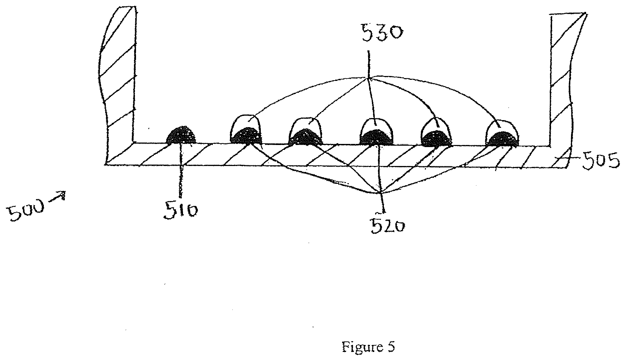

In another illustrative example, two or more capture antibody features can be printed on each well, and one or more calibration features of varying antigen concentrations can be printed on each well. Figure 5 shows a cross-sectional side view of one plate well with capture antibody features 510 and 520 printed at the bottom of the well 505. Five calibration features 530 with varying concentrations of antigen are precisely printed on top of capture antibody features 520. The series of calibration features with varying concentrations of antigen can be used to generate a standard curve. With the varying concentrations of the calibration features being known, the features produce detectable signals of varying intensity related to the known concentrations. The standard curve can be compared to the signal of the capture antibody feature binding to the antigen-containing test sample to determine the presence and quantity of the sample antigen. The disclosed method reduces or eliminates inaccuracy that can result from having the series of calibration features printed in separate wells from the capture antibody feature that will bind to the antigen-containing sample. It also results in increased throughput and efficiency of assays and other analyses.

The specific operational parameters provided above are merely illustrative, and other values are within the scope of the invention.

Kits can be made that incorporate the above devices along with any combination of related equipment or reagents, such as reporter reagents or software for reading results of the assay.The methods described above can be used to detect the presence of antigens and proteins in a patient, such as a patient having an autoimmune disease, antibodies to viral diseases, antibodies to bacterial diseases, antibodies to allergic reactions, or antibodies to cancers.

The terms and expressions that are employed herein are terms of description and not of limitation. There is no intention in the use of such terms and expressions of excluding the equivalents of the feature shown or described, or portions thereof, it being recognized that various modifications are possible within the scope of the invention as claimed.

## Claims

1. A method for detecting an antigen in a sample, the method comprising:
printing a plurality of capture antibody features in a well of a testing substrate,
printing a calibration compound of known concentration on one of the capture antibody features in the well of the testing substrate,
wherein the calibration compound of known concentration is capable of binding to the capture antibody feature,
wherein at least one capture antibody feature in the well of the testing substrate does not have a calibration compound of known concentration printed onto the at least one capture antibody feature and is capable of binding to the antigen in the sample, and
adding the sample to the well of the testing substrate,
wherein the calibration compound and the antigen are each capable of binding to a same enzyme linked detect antibody.

2. The method of claim 1, further comprising printing a plurality of calibration compounds on a respective plurality of capture antibody features, wherein the plurality of calibration compounds includes at least two different known concentrations of the calibration compound.

3. The method of claim 1 or claim 2, further comprising, prior to adding the sample to the well of the testing substrate:
incubating the printed testing substrate;
applying blocking material to the testing substrate;
drying the printed testing substrate; and
processing the printed testing substrate for usage or storage.

4. The method of claim 3, wherein the method is used to conduct biochemical analyses.

5. The method of claim 4, wherein the calibration compound that is capable of binding to the capture compound is an antigen.

6. The method of claim 5, wherein an enzyme-linked immunosorbent assay is conducted.

7. The method of claim 6, further comprising:
using the results from the at least two different concentrations of the calibration compound that is capable of binding to the capture antibody feature to create a calibration curve;
comparing the calibration curve to a signal of a capture antibody feature binding to an antigen-containing test sample; and
determining the presence and quantity of the antigen in the test sample.

## Patentansprüche

1. Verfahren zum Nachweis eines Antigens in einer Probe, wobei das Verfahren Folgendes umfasst:
Bedrucken einer Vielzahl von Einfangantikörpermerkmalen in einer Wanne eines Testsubstrats,
Drucken einer Kalibrierungskomponente bekannter Konzentration auf eine der Einfangantikörpermerkmale in der Wanne des Testsubstrats,
wobei die Kalibrierungskomponente bekannter Konzentration sich an das Einfangantikörpermerkmal binden kann,
wobei zumindest ein Einfangantikörpermerkmal in der Wanne des Testsubstrats keine auf das zumindest eine Einfangantikörpermerkmal aufgedruckte Kalibrierungskomponente bekannter Konzentration aufweist und sich an das Antigen in der Probe binden kann, und
Hinzufügen der Probe zu der Wanne des Testsubstrats,
wobei sich die Kalibrierungskomponente und das Antigen jeweils an denselben enzymverbunden Nachweisantikörper binden kann.

2. Verfahren nach Anspruch 1, ferner umfassend das Drucken einer Vielzahl von Kalibrierungskomponenten auf eine entsprechende Vielzahl von Einfangantikörpermerkmalen, wobei die Vielzahl von Kalibrierungskomponenten zumindest zwei verschiedene bekannte Konzentrationen der Kalibrierungskomponente einschließt.

3. Verfahren nach Anspruch 1 oder Anspruch 2, ferner Folgendes umfassend, vor dem Hinzufügen der Probe zu der Wanne des Testsubstrats:
Inkubieren des bedruckten Testsubstrats;
Aufbringen von Blockiermaterial auf das Testsubstrat;
Trocknen des bedruckten Testsubstrats; und
Verarbeiten des bedruckten Testsubstrats zur Verwendung oder Lagerung.

4. Verfahren nach Anspruch 3, wobei das Verfahren verwendet wird, um biochemische Analysen durchzuführen.

5. Verfahren nach Anspruch 4, wobei die Kalibrierungskomponente, die sich an die Einfangkomponente binden kann, ein Antigen ist.

6. Verfahren nach Anspruch 5, wobei ein Enzymimmunoassay durchgeführt wird.

7. Verfahren nach Anspruch 6, ferner umfassend:
Verwenden der Ergebnisse aus den zumindest zwei verschiedenen Konzentrationen der Kalibrierungskomponente, die sich an das Einfangantikörpermerkmal binden kann, um eine Kalibrierungskurve zu erzeugen;
Vergleichen der Kalibrierungskurve mit einem Signal eines Einfangantikörpermerkmals, das sich an eine antigenhaltige Testprobe bindet; und
Bestimmen des Vorhandenseins und der Menge des Antigens in der Testprobe.

## Revendications

1. Méthode de détection d'un antigène dans un échantillon, cette méthode comprenant :
l'impression d'une pluralité de caractéristiques d'anticorps de capture dans un puits d'un substrat d'essai,
l'impression d'un composé de calibrage à concentration connue sur une des caractéristiques d'anticorps de capture dans le puits du substrat d'essai,
le composé de calibrage d'une concentration connue étant capable de se lier à la caractéristique d'anticorps de capture,
au moins une caractéristique d'anticorps de capture dans le puits du substrat d'essai étant dépourvue de l'impression d'un composé de calibrage à concentration connue sur la caractéristique d'anticorps de capture au nombre d'au moins une, et étant en mesure de se lier à l'antigène dans l'échantillon, et
l'addition de l'échantillon au puits du substrat d'essai,
le composé de calibrage et l'antigène étant chacun capable de se lier à un même anticorps de détection lié à une enzyme.

2. Méthode selon la revendication 1, comprenant en outre l'impression d'une pluralité de composés de calibrage sur une pluralité respective de caractéristiques d'anticorps de capture, la pluralité de composés de calibrage comprenant au moins deux concentrations connues diverses du composé de calibrage.

3. Méthode selon la revendication 1 ou la revendication 2, comprenant également, avant l'addition de l'échantillon dans le puits de substrat d'essai :
l'incubation du substrat d'essai imprimé ;
l'application du matériau de blocage sur le substrat d'essai ;
le séchage du substrat d'essai imprimé ; et.
le traitement du substrat d'essai imprimé pour l'utilisation ou le stockage.

4. Méthode selon la revendication 3, la méthode étant utilisée pour effectuer des analyses biochimiques.

5. Méthode selon la revendication 4, le composé de calibrage capable de se lier au composé de capture étant un antigène.

6. Méthode selon la revendication 5, un dosage immunoenzymatique étant effectué.

7. Méthode selon la revendication 6, comprenant en outre :
l'utilisation des résultats des au moins deux concentrations diverses de composé de calibrage capables de se lier à la fonction d'anticorps de capture pour créer une courbe de calibrage ;
la comparaison de la courbe de calibrage avec un signal de la caractéristique d'anticorps de capture se liant à un échantillon d'essai contenant un antigène ; et
la détermination de la présence et de la quantité d'antigène dans l'échantillon d'essai.
